# EUROPÄISCHE PATENTSCHRIFT

(11) **EP 1 522 859 B1**
(45) Veröffentlichungstag und Bekanntmachung des Hinweises auf die Patenterteilung: **26.09.2012**
(21) Anmeldenummer: 04023728.1
(22) Anmeldetag: 05.10.2004
(51) Int. Cl.: G01N 33/86, G01N 31/22, G01N 33/487

(54) **On-Board-Kontrolle für Analyseelemente**
On-Board-Control for test elements
Contrôle intégré pour éléments d'analyse

(30) Priorität: 09.10.2003 DE 10346863
(43) Veröffentlichungstag der Anmeldung: 13.04.2005
(73) Patentinhaber: Roche Diagnostics GmbH, 68305 Mannheim (DE); F.Hoffmann-La Roche AG, 4070 Basel (CH)
(72) Erfinder: Unkrig, Volker, Dr., 68526 Ladenburg (DE); Nortmeyer, Christine, 68305 Mannheim (DE); Horn, Carina, Dr., 68647 Biblis (DE); Marquant, Michael, 68309 Mannheim (DE); Lungu, Mihail-Onoriu, Dr., 67365 Schwegenheim (DE); Hoenes, Joachim, Dr., 64673 Zwingenberg (DE); Kotzan, Holger, Dr., 68526 Ladenburg (DE); Dreibholz, Joerg, Dr., 67122 Altrip (DE)

(56) Entgegenhaltungen:
- DE-A- 2 941 471
- DE-A- 19 831 519
- US-A- 3 899 295
- US-A- 4 038 148
- US-A- 5 096 813
- US-A- 5 484 708

## Beschreibung

Die Erfindung betrifft ein Analyseelement, beispielsweise in Form eines Teststreifens, insbesondere eines Teststreifens zur Bestimmung eines Gerinnungsparameters, welches ein Kontrollreagenzsystem beinhaltet, das es erlaubt, funktionsfähige Analyseelemente von nicht funktionsfähigen Analyseelementen zu unterscheiden. Weiterhin betrifft die Erfindung entsprechende Verfahren zur Kontrolle von Analyseelementen.

Zur qualitativen und quantitativen Analyse von Bestandteilen einer flüssigen Probe, insbesondere einer Körperflüssigkeit von Menschen oder Tieren, werden zunehmend sogenannte trägergebundene Tests eingesetzt. Dabei werden Analyseelemente (auch bezeichnet als Testelemente) verwendet, bei denen mindestens ein Reagenz in einem aus einer oder mehreren Schichten bestehenden Testfeld eingebettet ist, das mit der Probe in Kontakt gebracht wird. Die Reaktion von Probe und Reagenz führt zu einer visuellen oder mit Hilfe eines Gerätes (meistens reflektionsphotometrisch oder elektrochemisch) auswertbaren Veränderungen des Analyseelementes. Nach der Durchführung eines Tests wird das benutzte Analyseelement entsorgt.

Es sind zahlreiche unterschiedliche Analyseelement-Typen bekannt, die sich durch Messprinzip (z. B. optisch oder elektrochemisch) und die verwendeten Reagenzien sowie durch ihren Aufbau, insbesondere hinsichtlich der Anordnung und Befestigung der Testschichten, unterscheiden. Von besonderer praktischer Bedeutung sind streifenförmige Analyseelemente. Diese auch als Teststreifen bezeichneten Analyseelemente bestehen im Wesentlichen aus einer länglichen Tragschicht aus einem Kunststoffmaterial, auf der ein oder mehrere Testfelder angebracht sind.

Die Analyseelemente werden in Primärverpackungen verpackt, in deren Innenräumen sie bis zum Verbrauch, d. h. der Entnahme durch den Benutzer und Durchführung eines Tests mit anschließender Entsorgung, lagern. Die Analyseelemente können dabei jeweils einzeln in ihrer eigenen Primärverpackung verpackt sein. Überwiegend sind Analyseelement-Packungseinheiten gebräuchlich, bei denen sich mehrere Analyseelemente im Innenraum einer gemeinsamen Primärverpackung befinden. In der Regel enthalten die Primärverpackungen ein Trockenmittel.

Der Innenraum der Primärverpackung ist üblicherweise weitgehend luftdicht abgeschlossen. Die Lagerungsbedingungen sind somit im Wesentlichen durch die Umweltbedingungen in der Primärverpackung während der Lagerung bestimmt.

Viele Analyseelemente enthalten Reagenzien, die infolge bestimmter Lagerungsbedingungen, z. B. Temperatur, Feuchtigkeit, Sauerstoff, Licht etc., Schaden nehmen können, sodass sie für die Durchführung eines zuverlässigen Tests unbrauchbar werden. Zur Vermeidung von Schäden ist es daher erforderlich, die Analyseelement-Packungseinheit unter bestimmten, vom Hersteller empfohlenen Lagerungsbedingungen sachgemäß zu lagern. Seitens des Herstellers wird die Haltbarkeit des Analyseelements bei sachgemäßer Lagerung für einen bestimmten Zeitraum garantiert.

Die Verwendung eines Analyseelements mit geschädigtem Reagenz kann zu einem falschen Testergebnis führen, woraus unter Umständen eine schwerwiegende Fehleinschätzung, beispielsweise des Gesundheitszustandes einer Person resultiert. Daher wurden in der Vergangenheit Anstrengungen unterschiedlicher Art unternommen, um die Gefahr der Verwendung von Analyseelementen mit Lagerungsschäden zu verringern.

Beispielsweise sind Testreagenzien entwickelt worden, die gegenüber äußeren Einflüssen relativ unempfindlich sind. Eine andere Entwicklung geht dahin, aufwendige Primärverpackungen zu verwenden, um die Einwirkungen äußerer Einflüsse auf die Reagenzien der Analyseelemente gering zu halten. Beide Lösungen sind mit wesentlich erhöhten Herstellungskosten verbunden. Aus Sicherheitsgründen wird oft ein relativ kurzer Haltbarkeitszeitraum angegeben. Dies führt dazu, dass Analyseelemente nach Ablauf des Haltbarkeitsdatums nicht mehr verwendet werden, wobei jedoch nicht nachprüfbar ist, ob tatsächlich Bedingungen geherrscht haben, die eine Schädigung der Reagenzien bewirkt haben könnten.

Teststreifen für diagnostische Blutuntersuchen unterliegen vor dem Vertrieb einer umfangreichen Qualitätskontrolle. Die geeigneten Versandbedingungen und Lagerbedingungen werden vor der Markteinführung intensiv untersucht und beispielsweise auf der Verpackung oder in der Packungsbeilage beschrieben. Trotzdem ist es nicht vollständig ausgeschlossen, dass beim Transport der Ware zum Kunden oder durch unsachgerechte Aufbewahrung beim Kunden Streifen vor dem Verfallsdatum geschädigt werden und die Gefahr besteht, dass bei ihrer Verwendung falsche Messwerte erhalten werden.

Unsachgemäße Transport- und/oder Lagerbedingungen können durch Messungen mit Flüssigkontrollen, die für die meisten dezentral eingesetzten (d.h. ausserhalb von spezialisierten Labors, also beispielsweise in Arztpraxen, Apotheken, oder beim Patienten zu Hause) sogenannten "Point-of-Care-Systeme" (PoC-Systeme) als weitere Systemkomponente neben Gerät und Streifen vertrieben werden, entdeckt werden. Nachteile der Verwendung von Flüssigkontrollen bei PoC-Systemen (z. B. für Gerinnungsmesssysteme) sind die nicht ganz einfache Handhabung, die Kosten für den Verbrauch von meist 2 Teststreifen und 2 Kontrollflüssigkeiten (Level 1 - & Level 2 - Kontrolle) sowie die Tatsache, dass bei den Kontrollmessungen zwar meist Streifen aus der selben Herstellcharge und Verpackung erfasst werden, dies allerdings zwangsläufig andere Streifen sind, als diejenigen, mit denen tatsächlich die Blutprobe eines Patienten untersucht wird.

Diese Nachteile vermeidet man mit einer "On-Board-Kontrolle" (englisch: On-board control, im Folgenden abgekürzt "OBC"), die in jedem Teststreifen integriert ist und ohne eine weitere Testflüssigkeit auskommt. Systeme mit "On-Board-Kontrollen" benötigen keine Kontrollflüssigkeiten, sondern arbeiten mit der selben Probenflüssigkeit, aus der der zu bestimmende Parameter mit dem Messsystem bestimmt wird.

Derartige "On-Board-Kontrollen" sind beispielsweise in den Patenten US 5,504,011 (ITC), US 6,084,660 (Lifescan) und US 6,060,323 (Hemosense) beschrieben. Den in den genannten Dokumenten offenbarten Kontrollsystemen ist gemeinsam, dass eine Blutprobe durch einen Kapillarkanal in ein Analyseelement aufgenomen wird und durch Kapillarkräfte zu einem Untersuchungsort innerhalb des Analyseelements transportiert wird. Durch eine oder mehrere Verzweigungen des Kapillarensystems wird dir Probe aufgeteilt und in einen oder mehrere Seitenkanäle geleitet. In diesen Seitenkanälen befinden sich dann Reagenzien, die die eigentliche OBC bilden.

Ein gemeinsamer Nachteil dieser "On-Board-Kontrollen" ist es, dass die Teststreifen - neben dem eigentlichen Messkanal für die Patienten probe - mehrere (meist zwei) weiterere Kanäle benötigen. In diesen zusätzlichen Kanälen werden nach Füllung mit demselben Patientenblut die Messungen durchgeführt, die Auskunft über die Integrität des Streifens erlauben sollen. Dieses Konzept führt zu hohen Herstellkosten, da die einzelnen Kanäle separat mit unterschiedlichen Reagenzien ausgestattet werden müssen. Weiterhin benötigen derartige Kontrollsysteme vergleichsweise hohen Probenvolumina, da neben dem eigentlichen Messkanal zumindest ein, meist jedoch sogar mehrere Kontrollkanäle mit Probe befüllt werden müssen. Hohe Probenvolumina werden insbesondere dort als nachteilig empfunden, wo Patienten selbst regelmäßig die Probe gewinnen müssen, also beispielsweise beim sogenannten "Home-Monitoring", insbesondere bei Diabetikern oder Patienten, die Gerinnungswerte selbst kontrollieren müssen, da die Gewinnung von Blutproben durch Punktur der Haut schmerzhaft ist und zwar umso schmerzhafter, je mehr Blutprobe benötigt wird. Darüberhinaus leiden Mehrkanal-On-Board-Kontrollsysteme unter schwierigen Befüllungsmechanismen, da die Probe selbständig in mehrere Kanäle eindringen und diese befüllen muss.

Aus DE-A 198 31 519 ist es bekannt, auf Testelementen oder deren Verpackung irreversible Feuchte- oder Temperaturindikatorelemente anzubringen, die eine Schädigung von Testelementen, beispielsweise durch zu hohe Temperaturen oder Luftfeuchtigkeit, durch Licht oder Sauerstoffeinwirkung, anzeigen sollen.

Die der Erfindung zugrundeliegende Aufgabe bestand darin, ein On-Board-Kontrollsystem zu entwickeln, das die genannten Nachteile - insbesondere bezüglich Herstellkosten und Probenvolumen - nicht besitzt und trotzdem zuverlässig die mögliche Unbrauchbarkeit von Testelementen, insbesondere aufgrund falscher Lager - oder Transportbedingungen, anzeigt. Insbesondere sollte schädigende Feuchtigkeits - und/oder Temperaturbelastung der Testelemente mit Hilfe der erfindungsgemäßen OBC erkannt werden. Die angestrebte OBC sollte desweiteren nicht zu früh reagieren, um eine lange Raumtemperaturlagerung des Produkts zu ermöglichen, und mit hoher Präzision eine gute Diskriminierung von intakten und defekten Streifen ermöglichen.

Diese Aufgabe wird durch den Gegenstand der Erfindung gelöst.

Der Gegenstand der Erfindung ist ein Analyseelement mit einem Reagenzsystem zur direkten oder indirekten Anzeige von Umweltbedingungen, die die Zuverlässigkeit des Analyseelements beeinträchtigen können, gemäß Anspruch 1, dessen Verwendung gemäß Anspruch 16, sowie ein Verfahren zur Kontrolle von Analyseelementen gemäß Anspruch 10. Vorteilhafte Ausgestaltungen der Erfindung sind Gegenstand der Unteransprüche.

Die Erfindung wird durch die nachfolgende detaillierte Beschreibung, die Figuren und Beispiele näher erläutert.

Das erfindungsgemäße Konzept, das auch als 1-Kanal-On-Board-Kontrolle bezeichnet werden kann, sieht vor, dass ein Reagenzbezirk auf einem Testelement, welcher vorzugsweise ebenfalls für die eigentliche Vermessung der Patientenprobe dient, eine Substanz enthält, die sich bei Belastung verändert. Die Veränderung dieser Substanz führt dabei bevorzugt zu einem Abbauprodukt, welches entweder visuell oder von dem für den Teststreifen zur Vermessung vorgesehenen Gerät detektiert wird. Ein als defekt erkannter Streifen kann beispielsweise durch das Messgerät für die Messung der Patientenprobe nicht frei gegeben werden.

Das erfindungsgemäße Analyseelement mit einem Reagenzsystem zur direkten oder indirekten Anzeige von Umweltbedingungen, die die Zuverlässigkeit des Analyseelements beeinträchtigen können eignet sich zur On-Board-Kontrolle von Analyseelementen. Das Reagenzsystem zur direkten oder indirekten Anzeige von Umweltbedingungen, die die Zuverlässigkeit des Analyseelements beeinträchtigen können, enthält dabei zumindest eine chemische Substanz, vorzugsweise jedoch ein Gemisch von chemischen Substanzen. Zumindest eine der Substanzen eignet sich dabei für die direkte oder indirekte Anzeige von Umweltbedingungen, die die Zuverlässigkeit eines Analyseelements beeinträchtigen können. Diese Eigenschaft wird als On-Board-Kontrolleigenschaft bezeichnet. Dabei werden schädigende Umwelteinflusse, wie Temperatur, Feuchtigkeit, Licht, Sauerstoff etc., dazu ausgenutzt, in der besagten Substanz oder mit der besagten Substanz eine vorzugsweise irreversible Veränderung durchzuführen, die eine nachträgliche Detektion der schädigenden Einflüsse ermöglicht. Hierbei ist es meist unerheblich, die genaue Art der schädigenden Einflüsse zu bestimmen; es reicht in aller Regel, die Tatsache nachzuweisen, dass ein schädigendes Ereignis stattgefunden hat.

Das Reagenzsystem zur On-Board-Kontrolle kann neben der Substanz, die die eigentliche On-Board-Kontrollfunktion bewirkt, weitere Hilfssubstanzen enthalten. Beispielsweise können dies Puffersubstanzen, Füllstoffe, Filmbildner und dergleichen mehr sein, die dem Fachmann in einer Vielzahl von Ausführungsformen im Zusammenhang mit Reagenzienrezepturen für analytische Testelemente bekannt sind.

Im erfindungsgemäßen Sinn sind Analyseelemente (auch als Testelemente, analytische Testelemente, Teststreifen, Testchips, Testdevices bezeichnet) vorzugsweise für die Bestimmung von Analyten oder sonstigen Parametern in flüssigen Proben, insbesondere in flüssigen Proben menschlichen Ursprungs, wie Blut, Serum, Plasma, Urin und dergleichen mehr geeignet. Analyseelemente im erfindungsgemäßen Sinn enthalten stets auf einem Trägermaterial ein Reagenz oder Reagenzsystem, welches in Abhängigkeit von dem zu untersuchenden Analyten in der Probe bzw. von der zu untersuchenden Eigenschaft der Probe ein detektierbares Signal erzeugen. Solche Analyseelemente sind dem Fachmann in einer Vielzahl von Ausführungsformen bekannt. Beispielsweise seien genannt optische oder elektrochemische Teststreifen für den Nachweis von Metaboliten in Blut oder davon abgeleiteten Probenflüssigkeiten, insbesondere zur Bestimmung von Glucose, Cholesterin und dergleichen. Darüber hinaus sind Teststreifen bekannt, mit deren Hilfe in einer Blutprobe Gerinnungsparameter bestimmt werden können.

Das Reagenzsystem, das auf dem Analysenelement angeordnet ist und zu einem detektierbaren Nachweissignal für einen Analyten in der Probe bzw. für eine Eigenschaft der Probe verantwortlich ist, kann vorzugsweise zu einem optisch erfassund auswertbaren Messsignal oder zu einem elektrochemisch detektierbaren Signal führen. Beide Varianten sind dem Fachmann in einer Vielzahl von Ausführungsformen bekannt.

Vorzugsweise ist das erfindungsgemäße Reagenzsystem zur On-Board-Kontrolle in das Reagenzsystem für die Nachweisreaktion integriert. Es ist jedoch auch denkbar und möglich, auf einem Analyseelement in räumlich getrennten Bezirken Reagenzsysteme für die Nachweisreaktion und Reagenzsysteme für die On-Board-Kontrolle unterzubringen.

Für den bevorzugten Fall, dass das Reagenzsystem für die On-Board-Kontrolle in das Reagenzsystem für die Nachweisreaktion integriert ist, ist darauf zu achten, dass die Reagenzien sich nicht gegenseitig negativ beeinflussen.

Das Reagenzsystems für die On-Board-Kontrolle und das Reagenzsystem für die Nachweisreaktion können auf den selben oder auf unterschiedlichen Nachweisprinzipien beruhen. So ist es erfindungsgemäß möglich, die On-Baord-Kontrolle optisch oder elektrochemisch auszuwerten. Ebenso ist es möglich, die eigentliche Nachweisreaktion optisch oder elektrochemisch zu erfassen. Besonders bevorzugt ist es, sowohl die On-Board-Kontrolle als auch die Nachweisreaktion elektrochemisch zu erfassen. Ein optischer Nachweis kann dabei visuell oder apparativ mit Hilfe eines Photometers erfolgen, wobei hier beliebige, dem Fachmann geläufige Techniken, wie Reflektionsmessung, Absorptionsmessung, Transmissionsmessung, Lumineszenzmessung und dergleichen mehr, eingesetzt werden können. Für die elektrochemische Detektion eignen sich wiederum sämtliche, dem Fachmann bekannte Techniken, wie Potentiometrie, Amperometrie, Coulometrie, Chronoamperometrie und dergleichen mehr.

Insbesondere die optisch auszuwertende Veränderung des On-Board-Kontrollfeldes bzw. des Nachweisreagenzienfeldes kann ohne Zuhilfenahme eines Messgeräts mit bloßem Auge durchgeführt werden. Bevorzugt ist es aber, die Auswertung der On-Baord-Kontrolle und des Nachweisfeldes mit Hilfe eines Messgeräts, beispielsweise eines Photometers oder eines elektrochemischen Messgeräts durchzuführen. Es ist auch möglich, die On-Board-Kontrolle ohne Messgerät, die Nachweisreaktion jedoch mit einem Messgerät auszuwerten.

Die Reagenzienrezepturen der On-Board-Kontrolle und des Nachweisfeldes können mit beliebigen, dem Fachmann bekannten Methoden auf einen Träger eines Analyseelementes aufgebracht werden. Dabei können die unterschiedlichen Reagenzien mit den selben oder mit unterschiedlichen Methoden aufgebracht werden. Beispielsweise seien als mögliche Methoden genannt: Aufbringen in flüssiger Form und anschließendes Auf- bzw. Eintrocknen auf bzw. in einen Träger; Aufbringen als Beschichtungsmasse durch Aufrakeln, Schlitzdüsenbeschichtung und dergleichen mehr; Druckverfahren wie Ink-Jet-Druck, Siebdruck, Dispergieren etc. Es ist auch möglich, bereits auf einen ersten Träger aufgebrachte Reagenzien mit diesem ersten Träger auf den eigentlichen, zweiten Träger des Analyseelements aufzubringen und mit diesem zu verbinden, z.B. durch Verkleben, Verschweissen etc.

Die erfindungsgemäße On-Board-Kontrolle soll zuverlässig belastete Testelemente erkennen können. Unter Belastung wird hierbei verstanden, dass die Testelemente Umweltbedingungen ausgesetzt werden, die zu einer Schädigung oder Beeinträchtigung der Reagenzien für die eigentliche Nachweisreaktion führen können. Für Analyseelemente, für die eine Raumtemperaturlagerung in geschlossenen, mit Trockenmittel versehenen Dosen oder für feuchtigkeitsundurchlässige Folien vorgesehen ist, ist unter Belastung beispielsweise das Ausgesetztsein gegenüber hohen Temperaturen in an sich geschlossenen Dosen oder Folien (z. B. beim Transport zum Kunden oder hinter einer Glasscheibe im Sonnenlicht) oder eine feuchte Lagerung (bei nicht sachgerecht verschlossenen Dosen nach Entnahme von Teststreifen oder bei defekten Folienverpackungen). Natürlich können auch Kombinationen von belastenden Ereignissen, wie beispielsweise gleichzeitige Feuchtigkeit oder erhöhte Temperatur eine Schädigung hervorrufen. Wichtig ist hier nur, dass eine Belastung schlussendlich zu einer Beeinträchtigung oder einem Versagen des Nachweisreagenzsystems führt. Für unterschiedliche Analyseelemente können dies durchaus unterschiedliche konkrete Bedingungen sein. Dem Fachmann ist bekannt, wie er entsprechende schädigende Ereignisse für das jeweilige Reagenzsystem identifizieren kann.

Erfindungsgemäß führt eine Belastung des Analyseelements bzw. des Reagenzsystems zur On-Board-Kontrolle zu einer - vorzugsweise irreversiblen - Veränderung des Reagenzsystems zur On-Board-Kontrolle. Somit ist gewährleistet, dass schädigende Umwelteinflüsse auch dann erkannt werden, wenn die Umweltbedingungen zwischenzeitlich sich in an sich günstige Bedingungen geändert haben sollten. Beispielsweise kann so eine einmalige schädigende Temperaturerhöhung oder eine einmalige kurzzeitige Einwirkung von Feuchte, die zu Schädigungen geführt hat, zuverlässig identifiziert werden.

Überraschenderweise wurde gefunden, dass Reagenzien, die N-Oxide oder Nitrosoverbindungen, insbesondere in Verbindung mit Reduktionsmitteln wie Zuckern, Polyalkoholen, cysteinhaltigen Proteinen, Glycin, etc., enthalten, unter denselben Bedingungen und Umwelteinflüssen reduziert werden, unter denen eine OBC unerwünschte Einflüsse und negative Veränderungen eines Teststreifens anzeigen sollte. Die durch Reduktion aus den N-Oxiden oder Nitrosoverbindungen entstandenen Abbauprodukte können im Teststreifen durch geeignete, vorzugsweise elektrochemische oder optische Verfahren detektiert werden. Insbesondere für den Fall, dass als Reduktionsmittel Zucker verwendet werden, können die Redoxeigenschaften und damit die Kinetik der Redoxreaktion über den pH-Wert des Reagenzes eingestellt werden.

Erfindungsgemäß hat sich als besonders gut geeignetes Molekül für die beschriebene "Indikatorreaktion" und damit als bevorzugt das N-Oxid Resazurin herausgestellt.

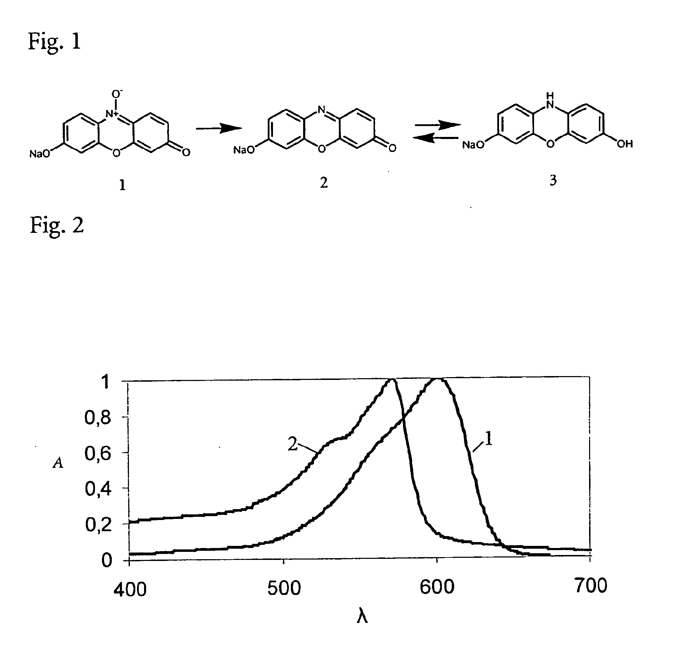

Das blaue Resazurin wird bei Belastung unter Bedingungen, die einen Teststreifen schädigen können (erhöhte Temperaturen, Feuchtigkeit und Licht), zu dem roten Resorufin reduziert (vgl. Fig. 1), wobei die Reduktion insbesondere in Gegenwart geeigneter Redoxpartner, vorzugsweise solcher aus der Reagenzienrezeptur, stattfindet. Die Veränderung, d.h. die Abnahme der Resazurinkonzentration bei gleichzeitiger Zunahme der Resorufinkonzentration, kann visuell, durch einen optischen Detektor in einem Messgerät oder durch einen elektrochemischen Sensor festgestellt werden.

Für Resazurin wurde überraschenderweise Glycin als sehr spezifisches Reduktionsmittel gefunden. Die Kombination von geeigneten Mengen Resazurin mit Glycin in einem OBC-Reagenz ist erfindungsgemäß besonders bevorzugt. Für Resazurin hat sich dabei eine Mindestkonzentration von ca. 0,01 g/l als bevorzugt herausgestellt, da unterhalb dieser Konzentration ein Nachweis des Resazurins praktisch sehr schwierig ist. Die Höchstmenge an Resazurin sollte 20 mmol/l nicht überschreiten, da ansonsten Löslichkeitsprobleme auftreten können. Wie oben beschrieben ist Glycin nicht unbedingt für das Funktionieren der OBC mit Resazurin erforderlich; als maximale Menge an Glycin hat sich eine Konzentration von ca. 250 g/l erwiesen, da oberhalb dieser Menge Löslichkeitsprobleme auftreten und Gylcin unter Umständen aus der Lösung auskristallisiert, was wiederum beim Beschichten der Reagenzmasse problematisch sein kann und möglicherweise zu Inhomogenitäten in der beschichteten Masse führen kann.

Für die elektrochemische Detektion der OBC-Reaktion kann die Quantifizierung des verbliebenen Resazurins und/oder die Quantifizierung des entstandenen Resorufins verwendet werden.

Die Quantifizierung von Resazurin ist bei großen Konzentrationsänderungen zu bevorzugen und kann beispielsweise durch elektrochemische Reduktion des Resazurins bei einem Potential von -700mV gegen Ag/AgCl durchgeführt werden. Bei diesem Potential wird das entstehende Resorufin bis zum Dihydro-Resorufin weiter reduziert (vgl. Fig. 1).

Wenn im OBC-Reagenz überwiegend Resazurin vorliegt (und das Reagenz folglich nicht oder kaum belastet ist) wird ein 4-Elektronenübergang provoziert, der zu einem höheren Strom führt als wenn vorwiegend Resorufin vorliegt, dessen Reduktion nur zu einem Umsatz von 2 Elektronen führt. Der bei einem vorgegebenen Potential, insbesondere bei dem bevorzugten Potential von -700mV gegen Ag/AgCl gemessene Strom bzw. Ladung erlauben somit Rückschlüsse auf die Höhe der Temperaturund/oder Feuchtigkeitsbelastung des Reagenzes

Die Quantifizierung von Resorufin ist durch elektrochemische (Re-)Oxidation des Resorufins zu Resazurin nicht möglich, weil die Reduktion von Resazurin zu Resorufin irreversibel verläuft. Bei einem reduktiven Nachweis in dem beschriebenen OBC-System ist es notwendig, spezifisch nur die Reduktion von Resorufin, nicht jedoch die Reduktion von eventuell im Reagenz vorhandenem Resazurin zu erfassen. Dies gelingt beispielsweise durch die Verwendung eines spezifischen Reduktionspotentials, welches bevorzugt im Bereich von -450 bis -550 mV gegen Ag/AgCl liegt.

Überraschenderweise wurde gefunden, dass die Quantifizierung von Resorufin in dem beschriebenen OBC-System besonders gut möglich ist, wenn in einem ersten Schritt das im Teststreifen vorliegende Resorufin elektrochemisch in Dihydro-Resorufin umgewandelt wird (im Folgenden "OBC Prepare" genannt) und in einem zweiten Schritt (im Folgenden "OBC Test" genannt) das in situ erzeugte Dihydro-Resorufin elektrochemisch zu Resorufin zurückoxidiert wird. Diese Oxidationsreaktion verläuft besonders spezifisch vorzugsweise bei einem Potential von -100 mV gegen Ag/AgCl.

Durch die Länge der "OBC Prepare Phase" kann die Intensität und Spezifität des "OBC Test Signals" gesteuert werden.

Neben dem besonders bevorzugten Resazurin/Resorufin-System sind Nitrosoverbindungen, insbesondere p-Nitrosoaniline, ein weiteres bevorzugtes Beispiel für eine Substanzklasse, die unter den Bedingungen, die Teststreifen schädigen können, zu Produkten umgesetzt werden können, die eine eventuelle Schädigung eines Teststreifens anzeigen können. p-Nitrosoaniline können beispielsweise unter den Bedingungen, die auch Reagenzien in einem Nachweisreagenz schädigen können, reduziert werden. Die reduktiv erzeugten Produkte (wie z.B. Phenylendiamine) können ebenfalls optisch oder elektrochemisch detektiert werden. Weitere Nitrosoverbindungen, die erfindungsgemäß eingesetzt werden können, sind in US 5,206,147, US 5,334,508, US 5,122,244 und US 5,286,362 beschrieben. Besonders bevorzugt ist eine Kombination der in diesen 4 US-Patenten offenbarten Nitrosoverbindungen mit Heteropolysäuren, insbesondere Heteropolysäuren in gefällter Form gemäß US 5,240,860, die bei Belastung in Gegenwart von Reduktionsmitteln leicht sichtbares Heteropolyblau bilden.

### Kurze Beschreibung der Figuren

Figur 1 zeigt die Strukturformeln von Resazurin 1, das durch Reduktion in Resorufin 2 und durch weitere Reduktion in Dihydro-Resorufin 3 umgewandelt wird.
Figur 2 zeigt Absorptionsspektren (relative Absorption A aufgetragen gegen die Wellenlänge λ (in nm)) von Resazurin 1 und Resorufin 2.
Figur 3 zeigt die Remissionsspektren (relative Remission R (in %) aufgetragen gegen die Wellenlänge λ (in nm)) der Reagenzfelder von unterschiedlich stark belasteten Testelementen (unbelastet 1, gering belastet 2, stark belastet 3).
Figur 4 zeigt anhand der Zunahme der relativen Remission R (in %) (gemessen bei einer Wellenlänge von 620 nm) über die Belastungszeit t (in h) die Zunahme an Abbauprodukt (Resorufin) in einem Resazurin-haltigen Reagenzfilm.
Figur 5 zeigt anhand der gemessenen Ladung Q in Abhängigkeit von der Belastungszeit t (in h) die Veränderung des elektrochemisch messbaren Signals während einer ersten, 3 Sekunden dauernden Messphase bei -700 mV vs. Ag/AgCl (Q₇₀₀) (untere beiden Kurven) und einer zweiten, 1,5 Sekunden dauernden Messphase bei -100 mV vs. Ag/AgCl (Q₁₀₀) (obere beiden Kurven).

Die Erfindung wird durch die nachfolgenden Beispiele näher charakterisiert. Dabei werden exemplarisch anhand von Teststreifen für die Gerinnungsmessung (am Beispiel eines Prothrombinzeittests oder PT-Tests) die Vorteile und Eigenschaften der erfindungsgemäßen OBC beschrieben. Für den Fachmann ist klar, dass die anhand des Beispiels der Gerinnungsteststreifen gemachten Aussagen ebenso für andere Teststreifenarten (insbesondere für solche zur optischen oder elektrochemischen Bestimmung von Blutglucose, Lipiden wie Cholesterin und HDL-Cholesterin, Triglyceriden, etc., zur Bestimmung anderer Gerinnungsparameter als PT wie z. B. aPTT, ACT, ECT, anti-Faktor Xa-Tests, aber auch für immunologische Testelemente, insbesondere optisch auswertbare Chromatographieteststreifen) gelten und auf diese übertragen werden können.

### Beispiel 1: 2 Reagenzrezepturen mit unterschiedlichen Reduktionsmitteln für eine optisch auswertbare On-Board-Kontrolle

**Tabelle 1**

| **Rezeptur für eine optisch auswertbare OBC mit Glycin als Reduktionsmittel** | | |
|---|---|---|
| **Chemikalie** | **Quelle** | **Konzentration** |
| Saccharose | Sigma | 3.2 g / dl |
| Mowiol 4/86 | Clariant GmbH | 1.3 g / dl |
| Keltrol F | Kelco | 2.98 g / ml |
| Glycin | Sigma | 1.35 g / dl |
| HEPES¹ | Sigma | 0.33 mg / ml |
| Polyethylenglykol PEG 3,350 | Sigma | 1.33 g /dl |
| Rinderserumalbumin | Sigma | 0.4 g / dl |
| Mega 8 | Sigma | 0.67 mg / ml |
| Resazurin | Riedel de Haen | 0.96 mg / ml |

| | | |
|---|---|---|
| ¹ HEPES: [4-(2-Hydroxyethyl)-piperazino]-ethansulfonsäure | | |

Die in Tabelle 1 aufgeführten Stoffe wurden homogen vermischt und mit NaOH auf einen pH-Wert von 7,4 eingestellt. Die so erhaltene Reaktionsmasse wurde als 20 mm breites und ca. 10 µm dickes Reagenzband auf einen reflektionsfotometrisch zu vermessenden Teststreifen beschichtet.

**Tabelle 2**

| **Rezeptur für eine optisch auswertbare OBC mit Glucose als Reduktionsmittel** | | | | |
|---|---|---|---|---|
| **pH der Rezeptur** | **7,5** | **9** | **10** | **11** |
| **Chemikalie** | **Einwaage in g** | | | |
| Polyvinylpyrrolidon-Lösung¹ | 7,09 | 7,09 | 7,09 | 7,09 |
| Glucose | 1,2 | 1,2 | 1,2 | 1,2 |
| HEPES² | 1,25 | -- | -- | -- |
| CHES³ | -- | 1,04 | 1,04 | -- |
| CAPS⁴ | -- | -- | -- | 1,11 |
| bidestilliertes Wasser | 7,72 | 7,93 | 7,93 | 7,86 |
| Keltrol F Lösung⁵ | 17,04 | 17,04 | 17,04 | 17,04 |
| BM Propiofan 70 D | 5,69 | 5,69 | 5,69 | 5,69 |
| Titandioxidaufschlämmung⁶ | 55,32 | 55,32 | 55,32 | 55,32 |
| Resazurin | 0,28 | 0,28 | 0,28 | 0,27 |
| Hexanol | 0,17 | 0,17 | 0,17 | 0,17 |
| Methoxy-2-propanol | 4,25 | 4,25 | 4,25 | 4,25 |

| | | | | |
|---|---|---|---|---|
| ¹Polyvinylpyrrolidon (PVP) wird in einer Menge von 40 g/l in Wasser eingestreut und ca. 30 min. nachgerührt. | | | | |
| ² HEPES: [4-(2-Hydroxyethyl)-piperazino)-ethansulfonsäure | | | | |
| ³ CHES: 2-(Cyclohexyamino)-ethansulfonsäure | | | | |
| ⁴ CAPS: 3-(Cyclohexylamino)-1-propansulfonsäure | | | | |
| ⁵ Keltrol wird in einer Menge von 6,8 g/l unter Rühren in Wasser eingestreut und mehrere Stunden nachgerührt. Um ein vollständiges Aufquellen sicherzustellen lässt man den Ansatz vor einer weiteren Verwendung über Nacht bei Raumtempertaur stehen. | | | | |
| ⁶ 15 g Titandioxid wird unter Rühren in 38 ml Wasser eingestreut und dann in einem Dissolverrührer bei hoher Rührgeschwindigkeit homogenisiert. | | | | |

Der pH-Wert der Reagenzienmasse wird jeweils mit Natronlauge auf den angegebenen Wert eingestellt.

Die in Tabelle 2 aufgeführten Stoffe wurden homogen vermischt. Die so erhaltene Reaktionsmasse wurde als 20 mm breites und ca. 10 µm dickes Reagenzband auf einen reflektionsfotometrisch zu vermessenden Teststreifen beschichtet.

Die Reaktionsgeschwindigkeit der OBC-Reaktion, d.h. die von den Umgebungsbedingungen abhängige Umwandlung von Resazurin in Resorufin, kann über den pH-Wert eingestellt werden. Je alkalischer der pH-Wert, desto schneller erfolgt diese Umwandlung und desto sensitiver ist somit die OBC.

### Beispiel 2: Massenspektroskopischer Nachweis des Abbauprodukts nach Belastung in der Reagenzienrezeptur gemäß Tabelle 1 aus Beispiel 1

Für die Teststreifen, für die eine Raumtemperaturlagerung in geschlossenen mit Trockenmittel versehenen Dosen oder für Feuchtigkeit undurchlässigen Folien vorgesehen ist, darf die Abbaureaktion unter diesen Lagerbedingenen nicht oder nur in sehr geringem Maße ablaufen.

Als "Fehllagerung" sind vor allem
- hohe Temperaturen in geschlossenen Dosen / Folien (z.B. beim Transport zum Kunden oder hinter einer Glasscheibe im Sonnenlicht), und
- feuchte Lagerung (nicht sachgerecht verschlossene Dosen nach Entnahme von Teststreifen oder defekte Folienverpackungen) von der On-Board-Kontrolle zu detektieren.

Diese Anforderungen wurden durch folgende Belastungsmodelle simuliert:
- Lagerung über mehrere Wochen bei 50°C in geschlossenen Dosen
- Lagerung über Stunden und mehrere Tage bei 50°C und erhöhter (50% / 75%) Luftfeuchtigkeit
- Lagerung über mehrere Tage bei geöffneter oder defekter Verpackung unter Umweltbedingungen der Klimazone 4 (30°C, 70% Luftfeuchtigkeit)

Das Massenspektrum einer belasteten Ragenzienrezeptur, die gemäß Tabelle 1 in Beispiel 1 hergestellt wurde zeigt, dass nach Belastung (6 Stunden bei 50 °C und 75 % relativer Luftfeuchtigkeit) einige Prozent des Resazurins in Resorufin umgesetzt waren (vgl. auch Figur 1). Neben dem Hauptpeak des Massenspektrums von Resazurin bei 228.17 Masseneinheiten ist der Hauptpeak des Resorufinspektrums (212,20 Masseneinheiten) vorhanden.

### Beispiel 3: Absorptionsspektren von Resazurin und Resorufin (Fig. 2)

Die in Figur 2 wiedergegebenen Absorptionsspektren von Resazurin 1 (einem blauen Farbstoff) und Resorufin 2 (einem roten Farbstoff) zeigen, dass prinzipiell eine optische Detektion (bspw. visuell oder reflektionsfotometrisch) der Umwandlung von Resazurin 1 in Resorufin 2, die - wie in Beispiel 2 gezeigt - bei der Belastung einer Reagenzienrezeptur erfolgt, möglich ist.

### Beispiel 4: Reflektionsspekten von unbelasteten und belasteten Teststreifen

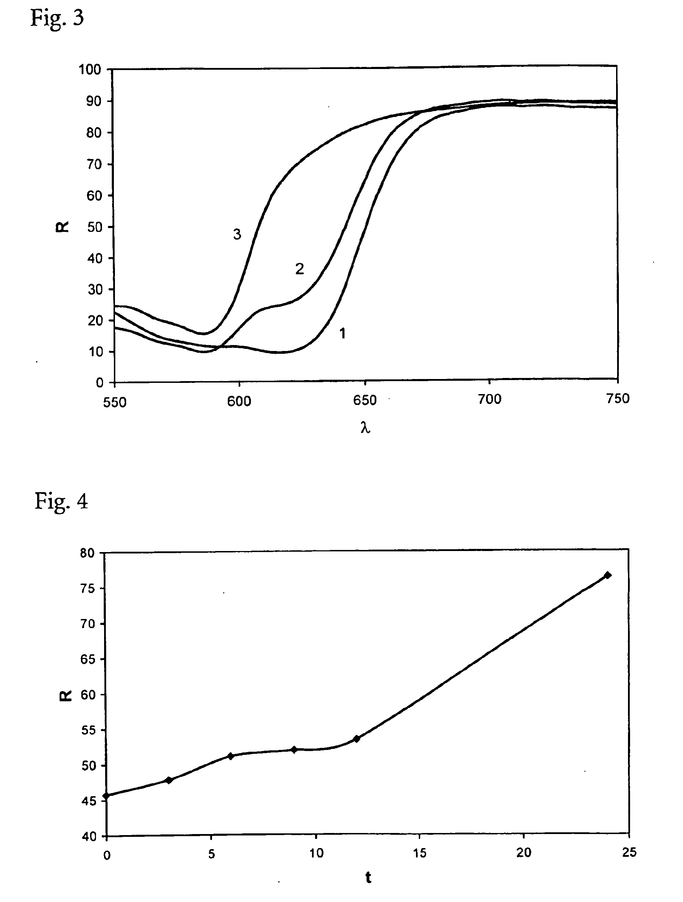

Teststreifen, deren Reagenzfilme auf Basis der Rezeptur aus Tabelle 1 von Beispiel 1 hergestellt waren, wurden 0 Stunden (unbelastet; vgl. Kurve 1 in Figur 3), 6 Stunden (gering belastet; vgl. Kurve 2 in Figur 3) und 12 Stunden (stark belastet; vgl. Kurve 3 in Figur 3) bei 50°C und 75% Luftfeuchtigkeit belastet. Die Veränderung der zugehörigen Remissionsspektren wurde gemessen (Fig. 3). Mit zunehmender Belastung zeigt sich eine Zunahme der Remission bei einer Wellenlänge 620 nm, die mit einer Abnahme der Menge an Resazurin verknüpft ist.

### Beispiel 5: Optische Detektion des Abbauprodukts Resorfurin in unterschiedlich stark belasteten Teststreifen

Teststreifen, deren Reagenzfilme auf Basis der Rezeptur aus Tabelle 1 von Beispiel 1 hergestellt waren, wurden unterschiedlich lange bei 50°C und 75% Luftfeuchtigkeit belastet und dann mit einem einfachen Reflektionsphotometer vermessen, dessen LED mit Licht der Wellenlänge 620 nm arbeitete.

Fig. 4 zeigt die Zunahme an Abbauprodukt (erkennbar an der Zunahme der Remission R) über die Belastungszeit t (in h).

Die Veränderung des Testfelds kann auch visuell, d.h. vom Benutzer mit bloßem Auge, leicht erkannt werden. Der zunächst blaue Reagenzbezirk des unbelasteten Teststreifens verändert seine Farbe mit zunehmender Belastungsdauer in rosa.

### Beispiel 6: Rezeptur für einen PT-Gerinnungstest mit integrierter OBC und elektrochemischer Detektion

**Tabelle 3**

| **Rezeptur für einen amperometrischen Prothrombinzeit-Test mit integrierten OBC-Reagenzien** | | |
|---|---|---|
| **Chemikalie** | **Quelle** | **Konzentration** |
| Relipidiertes rekombinates humanes Thromboplastin (rhTF) | Dade Behring, Marburg | 72 ng / ml |
| Saccharose | Sigma | 3.2 g / dl |
| Mowiol 4/86 | Clariant GmbH | 1.3 g / dl |
| Keltrol F | Kelco | 2.98 g / ml |
| Glycin | Sigma | 1.35 g / dl |
| Polybren | Sigma | 10 µg / ml |
| HEPES¹ | Sigma | 0.33 mg / ml |
| Polyethylenglykol PEG 3,350 | Sigma | 1.33 g / dl |
| Rinderserumalbumin | Sigma | 0.4 g / dl |
| Mega 8 | Sigma | 0.67 mg / ml |
| Resazurin | Riedel de Haen | 0.96 mg / ml |
| Electrozym TH | Roche Diagnostics | 1.2 mg / ml |

| | | |
|---|---|---|
| ¹HEPES: [4-(2-Hydroxyethyl)-piperazino]-ethansulfonsäure | | |

Die in Tabelle 3 aufgeführten Stoffe wurden homogen vermischt und mit NaOH auf einen pH-Wert von 7,4 eingestellt. Die so erhaltene Reaktionsmasse wurde in einer Breite von 4 mm und einer Dicke von ca. 90 µm (nass) bzw. ca. 10 µm (trocken) auf einen amperometrisch zu vermessenden Teststreifen beschichtet, so dass die Arbeitselektrode mit dem Reagenz vollflächig bedeckt war. Als Referenzelektrode diente eine Ag/AgCl-Elektrode, die ebenfalls als Gegenelektrode diente.

### Beispiel 7: Elektrochemische Detektion von Resazurin und des Abbauproduktions Resorfurin in unterschiedlich stark belasteten Teststreifen

Die gemäß Beispiel 6 hergestellten Teststreifen wurden unterschiedlich lange bei 50°C und 75% Luftfeuchtigkeit belastet. Mit diesen Teststreifen wurden dann Vollblute amperometrisch vermessen.

Für die Quantifizierung von Resazurin und Resorufin wurden folgende Potentiale angelegt:
-700 mV vs. Ag/AgCl für 3 Sekunden (sogenannte "OBC-Prepare"-Phase); anschließend
-100 mV vs. Ag/AgCl für 1,5 Sekunden (sogenannte "OBC-Test"-Phase); anschließend
+200 mV vs. Ag/AgCl für 90 Sekunden (für die eigentliche Gerinnungsmessung).

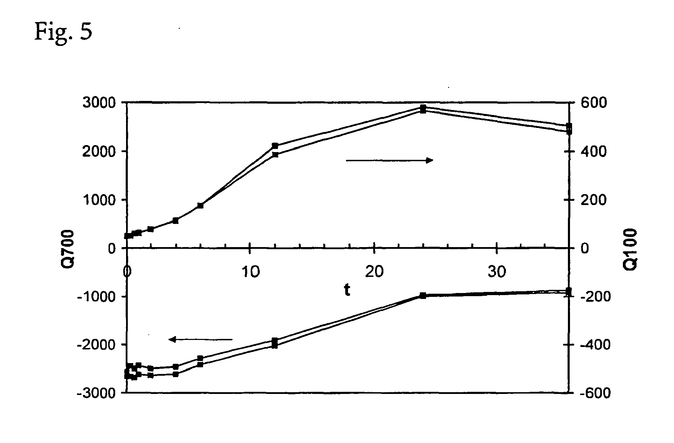

Für die Quantifizierung der "OBC Prepare" - und "OBC-Test" - Signale wurden die Integrale unter den Strom-Zeit-Kurven berechnet. Figur 5 zeigt, wie sich diese Integrale (als Q₇₀₀ bzw. Q₁₀₀ bezeichnet) mit zunehmender Belastung der Teststreifen verändern.

Während sich die OBC-Signale stark verändern, ist die mit belasteten Teststreifen gemessene Gerinnungszeit bis hin zu einer sehr langen Belastungsdauer nahezu stabil (vgl. Tabelle 4).

**Tabelle 4**

| **Auswirkungen einer Belastung bei 50°C und 75 % relativer Luftfeuchtigkeit auf die Messung der Gerinnungszeit** | |
|---|---|
| **Belastungszeit (h)** | **Gerinnungszeit (s)** |
| 0 | 12,78 |
| 0,33 | 12,38 |
| 0,66 | 12,72 |
| 1 | 13,14 |
| 2 | 12,52 |
| 4 | 13,53 |
| 6 | 13,26 |
| 12 | 12,78 |
| 24 | 13,16 |
| 36 | 14,00 |

Damit ist gewährleistet, dass die OBC die falsche Lagerung der Teststreifen anzeigt bevor falsche Gerinnungszeiten generiert werden.

### Beispiel 8: Anzeige der OBC mit elektrochemischer Detektion bei Lagerung unverpackter Streifen in Klimazone IV

Die größte Gefahr einer Schädigung von Tesstreifen, deren Verpackung defekt ist oder nicht wieder verschlossen wurde, besteht bei Kunden, die in der Klimazone IV leben (feucht warm). Für diese Klimazone werden in der Literatur als "mittlere Klimabedingungen" eine Feuchtigkeit von 70% und eine Temperatur von 30°C beschrieben.

Tabelle 5 zeigt, dass die OBC mit elektrochemischer Detektion Veränderungen des Teststreifens durch Lagerung bei diesen Bedingungen anzeigt.

Tabelle 5 zeigt für unterschiedliche Probenmaterialien (Normalblut (N) 1 und 2; Blut von Spendern, die mit Marcumar behandelt werden (M) 1 und 2), anhand der gemessenen Ladung Q (in nAs) in Abhängigkeit von der Belastungszeit t (in h) die Veränderung des elektrochemisch messbaren Signals während einer 3 Sekunden dauernden Messphase bei -700 mV vs. Ag/AgCl (Q₇₀₀) bzw. während einer 1,5 Sekunden dauernden Messphase bei -100 mV vs. Ag/AgCl (Q₁₀₀).

**Tabelle 5**

| **Auswirkungen einer Belastung bei 30°C und 70 % relativer Luftfeuchtigkeit auf die elektrochemische On-Board-Kontrolle für unterschiedliche Probenmaterialien** | | | | | | | | |
|---|---|---|---|---|---|---|---|---|
| **t (h)** | **Q₁₀₀ (nAs)** | | | | **Q₇₀₀ (nAs)** | | | |
| | N 1 | N 2 | M 1 | M 2 | N 1 | N 2 | M 1 | M 2 |
| 0 | 58,42 | 57,3 | 51,7 | 56,8 | 2663 | 2512 | 2580 | 2735 |
| 2 | 58,62 | 57,9 | 56,0 | 58,0 | 2779 | 2551 | 2619 | 2748 |
| 6 | 62 | 62,0 | 62,3 | 63,6 | 2673 | 2567 | 2568 | 2728 |
| 9 | 62,21 | 61,4 | 61,0 | 63,8 | 2644 | 2466 | 2535 | 2713 |
| 12 | 65,16 | 63,4 | 61,7 | 65,8 | 2637 | 2488 | 2480 | 2723 |
| 24 | 77,07 | 76,0 | 75,6 | 78,8 | 2608 | 2455 | 2485 | 2722 |
| 48 | 94,56 | 89,0 | 84,4 | 89,2 | 2622 | 2493 | 2526 | 2665 |
| 72 | 102,3 | 104,3 | 100,1 | 105,2 | 2615 | 2455 | 2545 | 2705 |
| 96 | 119 | 123,7 | 122,1 | 127,5 | 2575 | 2466 | 2479 | 2722 |
| 120 | 151 | 141,8 | 145,0 | 167,2 | 2491 | 2434 | 2467 | 2535 |

Sowohl anhand Messdaten, die während der sogenannten OBC-Prepare-Phase (vgl. Beispiel 7) als auch während der sogenannten OBC-Test-Phase (vgl. Beispiel 7) gewonnen werden, können somit "belastete" Analyseelemente identifiziert werden.

Die Gerinnungszeiten sind - wie Tabelle 6 zeigt - auch unter diesen Bedingungen deutlich stabiler, so dass belastete Teststreifen zuverlässig durch die OBC erkannt werden können, bevor unter Umständen falsche Gerinnungsmesswerte erzeugt worden wären.

**Tabelle 6**

| **Auswirkungen einer Belastung bei 30°C und 70 % relativer Luftfeuchtigkeit auf die Messung der Gerinnungszeit** | |
|---|---|
| **Belastungszeit (h)** | **Gerinnungszeit (s)** |
| 0 | 11,66 |
| 2 | 11,94 |
| 6 | 12,28 |
| 9 | 12,14 |
| 12 | 12,27 |
| 24 | 11,82 |
| 48 | 11,88 |
| 72 | 11,66 |
| 96 | 11,63 |
| 120 | 11,67 |

### Beispiel 9: Glycin als spezifischer Redoxpartner für Resazurin

Lagert man Testelemente, die die Rezeptur aus Beispiel 6 enthalten, bei 25°C über 48 Stunden, so verändert sich das Verhältnis von Resazurin zu Resorufin, weil wegen der "OBC-Reaktion" ersteres in letztes umgesetzt wird. Die folgende Tabelle 7 zeigt, dass die Reaktionsgeschwindigkeit dieser Reaktion von der An- oder Abwesenheit von Glycin beeinflusst wird. Eine OBC ist auch bei Abwesenheit von Glycin möglich; die Empfindlichkeit der OBC wird jedoch durch die Anwesenheit von Glycin deutlich erhöht.

**Tabelle 7**

| **Relative Menge Resorufin (%) in Bezug auf die Gesamtmenge an Resorufin und Resazurin in Abhängigkeit von der Belastungsdauer** | | |
|---|---|---|
| **Belastungsdauer (h)** | **Resorufin-Anteil (%)** | |
| | **Reagenz ohne Glycin** | **Reagenz mit Glycin** |
| 0 | 5 | 10 |
| 0,5 | 6 | 16 |
| 1 | 7 | 21 |
| 3 | 8 | 27 |
| 6 | 8 | 41 |
| 9 | 10 | 52 |
| 18 | 12 | 72 |
| 24 | 14 | 89 |
| 48 | 24 | 89 |

## Patentansprüche

1. Analyseelement enthaltend ein Reagenzsystem für eine Nachweisreaktion und ein Reagenzsystem zur direkten oder indirekten Anzeige von Umweltbedingungen, die die Zuverlässigkeit des Analyseelements beeinträchtigen können,
**dadurch gekennzeichnet, dass**
das Reagenzsystem zur direkten oder indirekten Anzeige von Umweltbedingungen, die die Zuverlässigkeit des Analyseelements beeinträchtigen können, ein organisches N-Oxid oder eine Nitrosoverbindung enthält.

2. Analyseelement gemäß Anspruch 1, **dadurch gekennzeichnet, dass** das Reagenzsystem zur direkten oder indirekten Anzeige von Umweltbedingungen, die die Zuverlässigkeit des Analyseelements beeinträchtigen können, bei Belastung eine Veränderung erfährt, die optisch oder elektrochemisch nachweisbar ist.

3. Analyseelement gemäß Anspruch 1 oder 2, **dadurch gekennzeichnet, dass** das Reagenzsystem zur direkten oder indirekten Anzeige von Umweltbedingungen, die die Zuverlässigkeit des Analyseelements beeinträchtigen können, ein Reduktionsmittel umfasst.

4. Analyseelement gemäß Anspruch 3, **dadurch gekennzeichnet, dass** das Reduktionsmittel ausgewählt ist aus der Gruppe umfassend Zucker, Polyalkohole, Glycin, und cysteinhaltige Proteine.

5. Analyseelement gemäß Anspruch 4, **dadurch gekennzeichnet, dass** das Reduktionsmittel Glycin oder Glucose ist.

6. Analyseelement gemäß einem der Ansprüche 1 bis 5, **dadurch gekennzeichnet, dass** das N-Oxid Resazurin ist.

7. Analyseelement gemäß einem der Ansprüche 1 bis 5, **dadurch gekennzeichnet, dass** die Nitrosoverbindung ein gegebenenfalls substituiertes p-Nitrosoanilin ist.

8. Analyseelement gemäß einem der Ansprüche 1 bis 7, **dadurch gekennzeichnet, dass** da Reagenzsystem zur direkten oder indirekten Anzeige von Umweltbedingungen, die die Zuverlässigkeit des Analyseelements beeinträchtigen können, in das Reagenzsystem für die Nachweisreaktion integriert ist.

9. Analyseelement gemäß einem der Ansprüche 1 bis 8, **dadurch gekennzeichnet, dass** das Reagenzsystem für die Nachweisreaktion Reagenzien zur Bestimmung von Gerinnungsparametern enthält.

10. Verfahren zur Kontrolle von Analyseelementen gemäß einem der Ansprüche 1 bis 9, wobei das Reagenzsystem zur direkten oder indirekten Anzeige von Umweltbedingungen, die die Zuverlässigkeit des Analyseelements beeinträchtigen können, mit Hilfe eines Messgeräts optisch oder elektrochemisch auf Veränderungen hin untersucht wird, die auf eine Belastung des Analyseelements hindeuten können.

11. Verfahren gemäß Anspruch 10, **dadurch gekennzeichnet, dass** das Messgerät Analyseelemente, bei denen eine Belastung detektiert wurde, nicht zur Vermessung einer Probenflüssigkeit mit Hilfe des Reagenzsystems für die Nachweisreaktion freigibt.

12. Verfahren gemäß Anspruch 10 oder 11, **dadurch gekennzeichnet, dass** sowohl die Untersuchung des Reagenzsystems zur direkten oder indirekten Anzeige von Umweltbedingungen, die die Zuverlässigkeit des Analyseelements beeinträchtigen können, als auch die Untersuchung des Reagenzsystems für die Nachweisreaktion optisch erfolgt.

13. Verfahren gemäß Anspruch 10 oder 11, **dadurch gekennzeichnet, dass** sowohl die Untersuchung des Reagenzsystems zur direkten oder indirekten Anzeige von Umweltbedingungen, die die Zuverlässigkeit des Analyseelements beeinträchtigen können, als auch die Untersuchung des Reagenzsystems für die Nachweisreaktion elektrochemisch erfolgt.

14. Verfahren gemäß Anspruch 10 oder 11, **dadurch gekennzeichnet, dass** die Untersuchung des Reagenzsystems zur direkten oder indirekten Anzeige von Umweltbedingungen, die die Zuverlässigkeit des Analyseelements beeinträchtigen können, optisch und die Untersuchung des Reagenzsystems für die Nachweisreaktion elektrochemisch erfolgt.

15. Verfahren gemäß Anspruch 10 oder 11, **dadurch gekennzeichnet, dass** die Untersuchung des Reagenzsystems zur direkten oder indirekten Anzeige von Umweltbedingungen, die die Zuverlässigkeit des Analyseelements beeinträchtigen können, elektrochemisch und die Untersuchung des Reagenzsystems für die Nachweisreaktion optisch erfolgt.

16. Verwendung eines Reagenzsystems, enthaltend ein organisches N-Oxid oder eine Nitrosoverbindung, zur direkten oder indirekten Anzeige von Umweltbedingungen, die die Zuverlässigkeit eines Analyseelements beeinträchtigen können.

## Claims

1. Analytical element containing a reagent system for a detection reaction and a reagent system for the direct or indirect indication of environmental conditions which can impair the reliability of the analytical element,
**characterized in that**
the reagent system for the direct or indirect indication of environmental conditions which can impair the reliability of the analytical element contains an organic N-oxide or a nitroso compound.

2. Analytical element according to claim 1, **characterized in that** the reagent system for the direct or indirect indication of environmental conditions which can impair the reliability of the analytical element undergoes a change when stressed which can be detected optically or electrochemically.

3. Analytical element according to claim 1 or 2, **characterized in that** the reagent system for the direct or indirect indication of environmental conditions which can impair the reliability of the analytical element comprises a reducing agent.

4. Analytical element according to claim 3, **characterized in that** the reducing agent is selected from the group comprising sugars, polyalcohols, glycine and cysteine-containing proteins.

5. Analytical element according to claim 4, **characterized in that** the reducing agent is glycine or glucose.

6. Analytical element according to one of the claims 1 to 5, **characterized in that** the N-oxide is resazurin.

7. Analytical element according to one of the claims 1 to 5, **characterized in that** the nitroso compound is an optionally substituted p-nitrosonaniline.

8. Analytical element according to one of the claims 1 to 7. **characterized in that** the reagent system for the direct or indirect indication of environmental conditions which can impair the reliability of the analytical element is integrated into the reagent system for the detection reaction.

9. Analytical element according to one of the claims 1 to 8, **characterized in that** the reagent system for the detection reaction contains reagents for determining coagulation parameters.

10. Method for checking analytical elements according to one of the claims 1 to 9, wherein the reagent system for the direct or indirect indication of environmental conditions which can impair the reliability of the analytical element, is examined optically or electrochemically for changes which can indicate a stress on the analytical element with the aid of a measuring device.

11. Method according to claim 10, **characterized in that** analytical elements in which a stress was detected are not released by the measuring device for measuring a sample liquid with the aid of the reagent system for the detection reaction.

12. Method according to claim 10 or 11, **characterized in that** the examination of the reagent system for the direct or indirect indication of environmental conditions which can impair the reliability of the analytical element as well as the examination of the reagent system for the detection reaction is carried out optically.

13. Method according to claim 10 or 11, **characterized in that** the examination of the reagent system for the direct or indirect indication of environmental conditions which can impair the reliability of the analytical element as well as the examination of the reagent system for the detection reaction is carried out electrochemically.

14. Method according to claim 10 or 11, **characterized in that** the examination of the reagent system for the direct or indirect indication of environmental conditions which can impair the reliability of the analytical element is carried out optically and the examination of the reagent system for the detection reaction is carried out electrochemically.

15. Method according to claim 10 or 11, **characterized in that** the examination of the reagent system for the direct or indirect indication of environmental conditions which can impair the reliability of the analytical element is carried out electrochemically and the examination of the reagent system for the detection reaction is carried out optically.

16. Use of a reagent system containing an organic N-oxide or a nitroso compound for the direct or indirect indication of environmental conditions which can impair the reliability of an analytical element.

## Revendications

1. Élément d'analyse contenant un système de réactifs pour une réaction de décèlement et un système de réactifs pour l'affichage direct ou indirect de conditions ambiantes qui peuvent influencer négativement la fiabilité de l'élément d'analyse,
**caractérisé en ce que**
le système de réactifs pour l'affichage direct ou indirect de conditions ambiantes qui peuvent influencer négativement la fiabilité de l'élément d'analyse contient un N-oxyde organique ou un composé nitroso.

2. Élément d'analyse selon la revendication 1, **caractérisé en ce que** le système de réactifs pour l'affichage direct ou indirect de conditions ambiantes qui peuvent influencer négativement la fiabilité de l'élément d'analyse subit une modification en cas de sollicitation, qui peut être décelée par voie optique ou par voie électrochimique.

3. Élément d'analyse selon la revendication 1 ou 2, **caractérisé en ce que** le système de réactifs pour l'affichage direct ou indirect de conditions ambiantes qui peuvent influencer négativement la fiabilité de l'élément d'analyse comprend un agent de réduction.

4. Élément d'analyse selon la revendication 3, **caractérisé en ce que** l'agent de réduction est choisi parmi le groupe comprenant des sucres, des polyalcools, de la glycine et des protéines contenant de la cystéine.

5. Élément d'analyse selon la revendication 4, **caractérisé en ce que** l'agent de réduction est la glycine ou le glucose.

6. Élément d'analyse selon l'une quelconque des revendications 1 à 5, **caractérisé en ce que** le N-oxyde est la résazurine.

7. Élément d'analyse selon l'une quelconque des revendications 1 à 5, **caractérisé en ce que** le composé nitroso représente une p-nitrosoaniline éventuellement substituée.

8. Élément d'analyse selon l'une quelconque des revendications 1 à 7, **caractérisé en ce que** le système de réactifs pour l'affichage direct ou indirect de conditions ambiantes qui peuvent influencer négativement la fiabilité de l'élément d'analyse est intégré dans le système de réactifs pour la réaction de décèlement.

9. Élément d'analyse selon l'une quelconque des revendications 1 à 8, **caractérisé en ce que** le système de réactifs pour la réaction de décèlement contient des réactifs pour la détermination des paramètres de coagulation.

10. Procédé pour le réglage d'éléments d'analyse selon l'une quelconque des revendications 1 à 9, dans lequel le système de réactifs pour l'affichage direct ou indirect de conditions ambiantes qui peuvent influencer négativement la fiabilité de l'élément d'analyse fait l'objet d'un examen optique ou électrochimique à l'aide d'un appareil de mesure visant des modifications qui peuvent suggérer une sollicitation de l'élément d'analyse.

11. Procédé selon la revendication 10, **caractérisé en ce que** l'appareil de mesure ne libère pas des éléments d'analyse pour lesquels une sollicitation a été détectée pour le mesurage d'un liquide échantillon à l'aide du système de réactifs pour la réaction de décèlement.

12. Procédé selon la revendication 10 ou 11, **caractérisé en ce que**, aussi bien l'examen du système de réactifs pour l'affichage direct ou indirect de conditions ambiantes qui peuvent influencer négativement la fiabilité de l'élément d'analyse que l'examen du système de réactifs pour la réaction de décèlement ont lieu par voie optique.

13. Procédé selon la revendication 10 ou 11, **caractérisé en ce que**, aussi bien l'examen du système de réactifs pour l'affichage direct ou indirect de conditions ambiantes qui peuvent influencer négativement la fiabilité de l'élément d'analyse que l'examen du système de réactifs pour la réaction de décèlement ont lieu par voie électrochimique.

14. Procédé selon la revendication 10 ou 11, **caractérisé en ce que** l'examen du système de réactifs pour l'affichage direct ou indirect de conditions ambiantes qui peuvent influencer négativement la fiabilité de l'élément d'analyse a lieu par voie optique et l'examen du système de réactifs pour la réaction de décèlement a lieu par voie électrochimique.

15. Procédé selon la revendication 10 ou 11, **caractérisé en ce que** l'examen du système de réactifs pour l'affichage direct ou indirect de conditions ambiantes qui peuvent influencer négativement la fiabilité de l'élément d'analyse a lieu par voie électrochimique et l'examen du système de réactifs pour la réaction de décèlement a lieu par voie optique.

16. Utilisation d'un système de réactifs contenant un N-oxyde organique ou un composé nitroso pour l'affichage direct ou indirect de conditions ambiantes qui peuvent influencer négativement la fiabilité d'un élément d'analyse.
